# EUROPEAN PATENT APPLICATION

(11) **EP 4 016 449 A1**
(43) Date of publication of application: **22.06.2022**
(21) Application number: 20290082.5
(22) Date of filing: 16.12.2020
(51) Int. Cl.: G06T 7/00, A61B 5/02, A61B 6/00

(54) **AN ANGIOGRAM FOR STENOSIS ANALYSIS**

(71) Applicant: Koninklijke Philips N.V., 5656 AG Eindhoven (NL)
(72) Inventor: Auvray, Vincent Maurice André, 5656 AE Eindhoven (NL); Florent, Raoul, 5656 AE Eindhoven (NL); Raynaud, Caroline Denise Francoise, 5656 AE Eindhoven (NL)
(74) Representative: Philips Intellectual Property & Standards

(57) **Abstract**

The present invention relates to stenosis evaluation. In order to facilitate the selection of suitable images, a device (10) for selecting an angiogram for stenosis analysis is provided that comprises an image sequence supply (12), a data processor (14) and an output (16). The image sequence supply is configured to provide a sequence of image frames representative of a region of interest of a vascular structure comprising one stenosis. The data processor selects an image frame of the sequence of image frames that leads to best matching stenosis related measurements. The selection is based at least indirectly on at least one reference, and the best matching relates at least indirectly to a maximized accuracy of the measurements. The output provides the selected image frame. Thus, a sequence of image frames is used for calculating a predetermined stenosis related measurement and the results are compared to a reference. The image frame with the best matching result is selected for further stenosis analysis and documentation purposes.

## Description

### FIELD OF THE INVENTION

The present invention relates to stenosis evaluation, and relates in particular to a device for selecting an angiogram for stenosis analysis, to a system for stenosis analysis and to a method for selecting an angiogram for stenosis analysis.

### BACKGROUND OF THE INVENTION

An example of vascular related diseases are stenoses, i.e. a portion of the vessel with a reduced inner volume. Several possible treatments for stenosis including e.g. stenting are known. In order to find the appropriate treatment, an exact knowledge in form of stenosis analysis is necessary, such as quantitative coronary analysis (QCA). As an example WO 2017/016885 A1 relates to automatic pre and post quantitative coronary angiography. A basis for the analysis in QCA is often a frame, i.e. an image from a sequence of images that is suitable for the further steps. As an example, the user first has to select the proper angiographic sequence, and then the frame of this sequence that is optimal to perform the analysis of the stenosis of interest. However it has been shown that this can be cumbersome for the user, i.e. the clinician.

### SUMMARY OF THE INVENTION

There may thus be a need to facilitate the selection of suitable images.

The object of the present invention is solved by the subject-matter of the independent claims; further embodiments are incorporated in the dependent claims. It should be noted that the following described aspects of the invention apply also for the device for selecting an angiogram for stenosis analysis, for the system for stenosis analysis and for the method for selecting an angiogram for stenosis analysis.

According to a first aspect, a device for selecting an angiogram for stenosis analysis is provided. The device comprises an image sequence supply, a data processor and an output. The image sequence supply is configured to provide a sequence of image frames representative of a region of interest of a vascular structure comprising one stenosis. The data processor is configured to select an image frame of the sequence of image frames that leads to best matching stenosis related measurements. The selecting is based at least indirectly on at least one reference. The best matching relates at least indirectly to a maximized accuracy of the measurements. The output is configured to provide the selected image frame.

This provides the image of the sequence that will allow the best subsequent analysis. The optimal and thus selected image shows the stenosis with as little cluttering as possible, as little foreshortening as possible, and also as strong contrast and sharpness as possible. Since the selection is based on the calculation results, the selection is performed without having issued explicit rules allowing to perform that selection per se. The exact definition of each of these criteria above can be left unclear, and their relative importance does not need to be weighted. Contrary, determining whether a frame presenting a cluttered yet well-contrasted stenosis is preferred to a frame showing a faint yet non-cluttered stenosis, is not determined in an absolute sense, but replaced or mimicked by the so-to-speak calculations that were trained by being compared in their results.

After being trained, i.e. in the product as used after initialization, i.e. training the AI, result comparison is not performed in an example. The above described comparison objective is used in the training phase.

For example, the selection is advantageously applied in the context of X-ray systems for vascular stenting interventions.

As an effect of the selected frame, the practical evaluation of the QCA is accelerated and thus the extraction of the metrics. In a degraded yet appreciable version, the choice of the sequence of interest is also provided and then the frame of interest in the selected sequence is selected - while, for example, still asking the clinician to click on the stenosis.

The quantitative analysis of the stenoses as visible in the angiograms yields a series of metrics that allow the clinician to analyze, discuss and document an exam.

Most algorithms analyzing X-ray sequences for stenosis characterization start with the selection of one frame, over which the analysis is then performed. The crucial aspect to select the image of the sequence where the stenosis is best visible, ideally the one that will allow the best subsequent analysis, is thus provided meaning that the clinician is supported, since the analysis of stenosed is facilitated.

In an option, a device for selecting an angiogram for stenosis analysis is provided comprising an image sequence supply, a data processor and an output. The image sequence supply is configured to provide a sequence of image frames representative of a region of interest of a vascular structure comprising one stenosis. The data processor comprises a machine learning algorithm. The data processor is configured to operate in a first algorithm training mode and in a second use mode.

In the training mode, training on data with ground truth is provided, via frame selection by an expert or via reference measurement by an expert.

Once all that is done, the algorithm, i.e. the network structure and trained weights, is frozen and is ready to use without associated ground truth. In the use mode no comparison with any predetermined frame is performed. If successfully trained, the trained network with machine learning will provided results as good as the experts that built the ground truth.

In the use mode, the frame best suited for future stenosis measurements is found, i.e. found automatically. In the use mode, each frame passes through the network that will perform a large variety of convolutions over each frame and issue one quality measure for each frame. At that point, the image with the highest score is kept.

In an example, the machine learning algorithm is provided as an artificial intelligence algorithm. In an example, the machine learning algorithm is provided as a deep learning algorithm.

According to an example, for selecting the image frame, the data processor comprises a machine learning algorithm that is configured to be trained with training datasets comprising pre-selected exemplary image frames comprising a stenosis along with some ground truth comprising at least one of the group of: preferred frame number as selected by an expert or reference measure.

In an example, the machine learning algorithm has not been trained yet. In another example, the machine learning algorithm has already been trained as explained and is ready for use.

In an example, the selection is based on at least one reference, and the best matching relates to a maximized accuracy of the measurements to build the ground truth over which the training is then performed.

According to an example, for selecting the image frame, the data processor with a trained machine learning algorithm is configured to provide the selection without further ground truth and/or further comparison with a predetermined reference.

According to an example, for training of the selecting the image frame, the data processor is configured to compute a predetermined stenosis-related measurement for at least two of the image frames of the sequence of images. The data processor is also configured to compare the computed measurements with a reference measurement and to determine one of the computed measurements that is best matching with the reference measurement.

It is noted that this relates to the training phase. In the product, no direct reference is provided, so everything runs automatically in an example.

For the training of the algorithm, the ground truth is determined, over which the training is then performed.

In an option, the output is configured to select the best matching image frame.

According to an example, the data processor is configured to determine a region of interest comprising the stenosis before the predetermined stenosis-related measurement is computed.

According to an example, the data processor is configured to perform, for the selected image frame, predetermined metrics measurements relating to the stenosis. The output is configured to provide the measurements.

According to an example, the data processor is configured to segment at least the part of the vascular structure of the selected image frame which part comprises the stenosis.

According to a second aspect, a system for stenosis analysis is provided. The system comprises a device for selecting an angiogram for stenosis analysis according to one of the preceding examples. The system further comprises an X-ray imaging arrangement. The X-ray imaging arrangement is configured to provide the sequence of image frames.

According to a third aspect, a method for selecting an angiogram for stenosis analysis is provided. The method comprises the following steps: providing a sequence of image frames representative of a region of interest of a vascular structure comprising one stenosis; selecting an image frame of the sequence of image frames that leads to best matching stenosis related measurements, the selecting being based at least indirectly on at least one reference and the best matching relates at least indirectly to a maximized accuracy of the measurements; and providing the selected image frame.

According to an example, for selecting the image frame, a machine learning algorithm is trained with training datasets comprising pre-selected exemplary image frames comprising a stenosis along with some ground truth comprising at least one of the group of: preferred frame number as selected by an expert or reference measure.

According to an example, for selecting the image frame, the trained machine learning algorithm is providing the selection without further ground truth and/or further comparison with a predetermined reference.

According to an example, for training of the selecting the image frame it is provided the steps of: computing a predetermined stenosis-related measurement for at least two of the image frames of the sequence of images; comparing the computed measurements with a reference measurement and determining one of the computed measurements that is best matching with the reference measurement; and
In an option, selecting the best matching image frame is provided.

In an example, before computing the predetermined stenosis-related measurement, a region of interest is determined comprising the stenosis.

It is noted that when a machine learning algorithm is provided and discussed in the present context, it also referred to providing the machine learning algorithm as a deep learning algorithm

According to an aspect, for training, a sequence of image frames is used for calculating a predetermined stenosis related measurement and the results are compared to a reference. The image frame with the best matching result is selected for further stenosis analysis and documentation purposes.

The algorithm takes as input one sequence and a region of interest that can be large, but separates the stenosis of interest from the others (if any). It outputs the image of the sequence that will guarantee the stenosis quantification closest to the truth. In an example, the invention proposes a framework to automatically perform that selection, locally and without explicit formulation of metrics of quality.

In an example, a set of representative clinical sequences is prepared, each associated with a rough indication of the region of interest. The preferred image is selected on each of them, with the help of experts. This selection can be performed directly, each user having an excellent intuition on which image he/she sees the stenosis best - and consequently the automatic algorithm will perform best.

In an option, an atomization of the selection of a best frame inside an already selected sequence is provided.

According to an aspect, it is relied on an algorithm that gets trained off-line on exemplary data for which the ground truth (GT) is provided, e.g. selected frame, or reference QCA measure. The algorithm is then is applied on-line on new data for which no ground truth is available. In an aspect, the preferred frame is determined based on a comparison with some reference (attached to the considered sequence). Once the algorithm is used, in an example, some reference is provided with new data.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for selecting an angiogram for stenosis analysis.
Fig. 2 schematically shows an example of a system for stenosis analysis.
Fig. 3 shows basic steps of an example of a method for selecting an angiogram for stenosis analysis.
Fig. 4 shows steps of a further example of the method for selecting an angiogram for stenosis analysis of Fig. 3.
Fig. 5 shows an example of an angiogram for quantitative coronary analysis.
Fig. 6 shows an example of three different images of the same angiographic sequence.
Fig. 7 shows a further example of a framework for selecting an angiogram for stenosis analysis.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

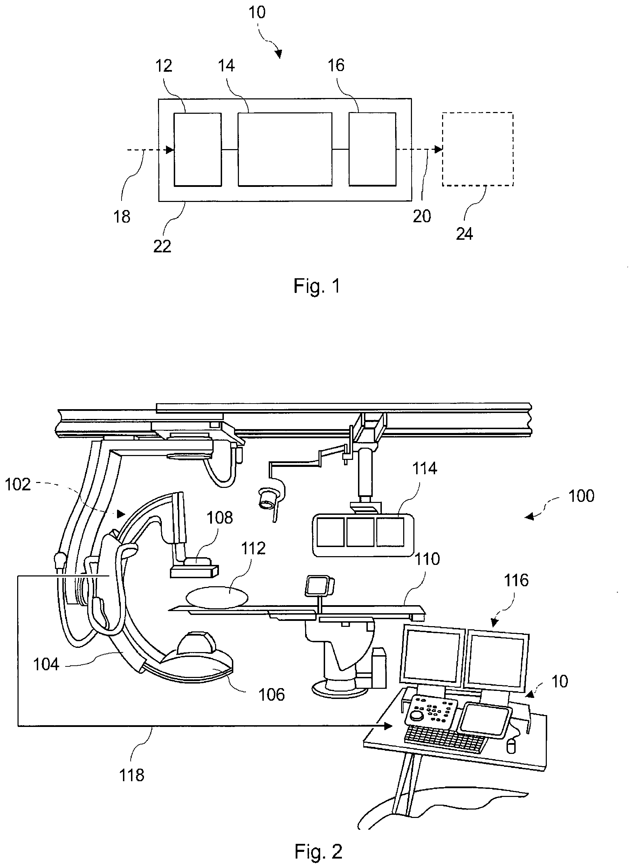

Fig. 1 schematically shows an example of a device 10 for selecting an angiogram for stenosis analysis. The device 10 comprises an image sequence supply 12, a data processor 14 and an output 16. The image sequence supply 12 is configured to provide a sequence of image frames representative of a region of interest of a vascular structure comprising one stenosis. The data processor 14 is configured to select an image frame of the sequence of image frames that leads to best matching stenosis related measurements. The selecting is based at least indirectly on at least one reference. Further, the best matching relates at least indirectly to a maximized accuracy of the measurements. The output 16 is configured to provide the selected image frame.

The term "based at least indirectly" relates to an example of a two-level approach described below. The reference is used in a first level that is provided to train the device. In the application, after being trained, the device performs without the direct use of the reference. However, since the training is based on the reference, also the trained application thus uses the reference, however, indirectly or implicit.

The term "relates at least indirectly" refers to this aspect of accurate measurements being provided in the training phase. The application then relies on this training phase.

A first hashed arrow 18 indicates the provision of the sequence of image frames, for example from a database or an image acquisition arrangement (both not shown). Further, a second hashed arrow 20 indicates the provision of the selected image frame.

In an example, the best matching relates to a maximized accuracy of the measurements to build the ground truth over which the training is then performed.

In an example, the image sequence supply 12, the data processor 14 and the output 16 are arranged in a common housing 22. In a further option, the image sequence supply 12, the data processor 14 and the output 16 are arranged as separate components data-connected with each other.

A hashed frame 24 indicates further data processing, data storage or data presentation such as provided by a display or graphical user interface, for further use of the selected image frame.

In an example, not further shown in detail, for selecting the image frame, the data processor 14 comprises a machine learning algorithm that is configured to be trained with training datasets comprising pre-selected exemplary image frames comprising a stenosis along with some ground truth comprising at least one of the group of: preferred frame number as selected by an expert or reference measure.

In an example, also not further shown in detail, for selecting the image frame, the data processor 14 with a trained machine learning algorithm is configured to provide the selection without further ground truth and/or further comparison with a predetermined reference.

In an example, not further shown in detail, for training of the selecting the image frame, the data processor 14 is configured to compute a predetermined stenosis-related measurement for at least two of the image frames of the sequence of images. The data processor 14 is also configured to compare the computed measurements with a reference measurement and to determine one of the computed measurements that is best matching with the reference measurement.

The output 16 is configured to select the best matching image frame.

The term "to compute" a predetermined stenosis-related measurement can also be referred to as to calculate the predetermined stenosis-related measurement.

In an example, the reference measurement is provided as a manually selected image.

In another example, the reference measurement is provided as a test-measurement image. The test-measurement image is an image set up with known geometric stenosis properties and thus being able to test the measurement results.

In an example, not further shown in detail, the data processor 14 is configured to determine a region of interest comprising the stenosis before the predetermined stenosis-related measurement is computed.

In an example, not further shown in detail, the data processor 14 is configured to perform, for the selected image frame, predetermined metrics measurements relating to the stenosis. Further, the output 16 is configured to provide the measurements.

In an example, not further shown in detail, the data processor 14 is configured to segment at least the part of the vascular structure of the selected image frame which part comprises the stenosis.

Fig. 2 schematically shows an example of a system 100 for stenosis analysis. The system 100 comprises an example of the device 10 for selecting an angiogram for stenosis analysis according to one of the examples described above and below. The system 100 also comprises an X-ray imaging arrangement 102. The X-ray imaging arrangement 102 is configured to provide the sequence of image frames. As an example, the X-ray image arrangement 102 is provided as a mobile C-arm system 104 with a movably mounted C-arm. An X-ray source 106 and an X-ray detector 108 are mounted to opposing ends of the C-arm. The C-arm may be suspended from a ceiling mount comprising rails. Further, a subject support 110 is provided in Fig. 2 for arranging a subject 112 for imaging and intervention purposes. A display arrangement 114 is shown in the vicinity of the subject support 110. The device 10 for selecting an angiogram for stenosis analysis is provided in form of a console arrangement 116 shown in the right foreground with monitors, interface elements like keyboard, mouse, graphic tablet and a control panel. The console arrangement 116, e.g. the device 10 for selecting an angiogram for stenosis analysis, is data-connected to the X-ray imaging arrangement 102, as indicated with data connection 118.

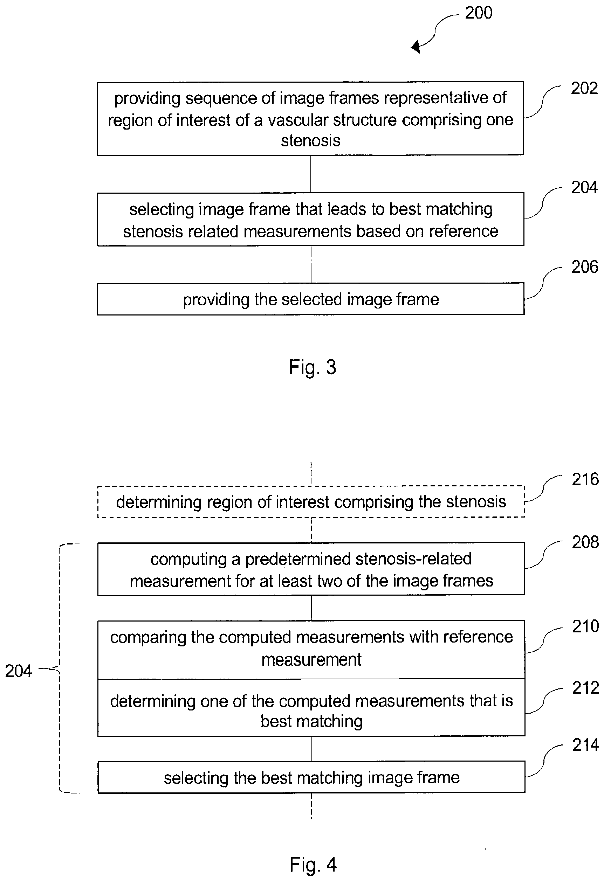

Fig. 3 shows basic steps of an example of a method 200 for selecting an angiogram for stenosis analysis. The method 200 comprises the following steps:
- In a first step 202, a sequence of image frames representative of a region of interest of a vascular structure comprising one stenosis is provided.
- In a second step 204, an image frame of the sequence of image frames is selected that leads to best matching stenosis related measurements. The selecting is based at least indirectly on at least one reference; and the best matching relates at least indirectly to a maximized accuracy of the measurements.
- In a third step 206, the selected image frame is provided.

The sequence comprises images of the vascular structure that is contrast injected at least in the region of the stenosis.

In an example, the measurement is provided as QCA, e.g. providing a ratio of the diameter of the stenosis before and after an interventional procedure such as stenting. The term maximized accuracy relates to the image based measurement in comparison to the actual value in the real world.

As a result, the crucial task to select the image of the sequence that will allow the best subsequent analysis is provided by the system. Instead of selecting the frame where the stenosis is best visible, the selection is provided focusing on the subsequent analysis. This may result in selecting a frame where the stenosis seems less visible from the perspective of a subjective observer or a frame that is also showing best visibility.

In a further example of the method 200, not shown in detail, for selecting the image frame, a machine learning algorithm is trained with training datasets comprising pre-selected exemplary image frames comprising a stenosis along with some ground truth comprising at least one of the group of: preferred frame number as selected by an expert or reference measure

In a further example of the method 200, not shown in detail, for selecting the image frame, the trained machine learning algorithm is providing the selection without further ground truth and/or further comparison with a predetermined reference.

Fig. 4 shows steps of a further example of the method 200 for selecting an angiogram for stenosis analysis of Fig. 3. As an example, for training of the selecting the image frame, i.e. for the second step 204, it is provided the following steps.
- In a first step 208, a predetermined stenosis-related measurement is computed for at least two of the image frames of the sequence of images.
- As a second step it is provided a first sub-step 210, in which the computed measurements are compared with a reference measurement a. In a second sub-step 212, one of the computed measurements that is best matching with the reference measurement is determined.
- In a further optional step 214, the best matching image frame is selected.

Providing the calculation of measurements for the frames to make the appropriate selection replaces the otherwise needed user experience. It is noted that it is rather difficult if not impossible to describe the criteria according to which the user makes an experience-based selection. The user assesses different criteria when evaluating the quality of each image in the context of stenosis analysis and weighs their relative importance. The optimal image should show the stenosis with, for example, as little cluttering as possible, as little foreshortening as possible, as strong contrast and sharpness as possible. Further, the selection has to be performed locally, with respect to one given stenosis.

In an example of the method 200, for selecting the image frame it is provided the steps of: providing a machine learning algorithm; and providing training datasets comprising pre-selected exemplary image frames comprising a stenosis.

The training datasets may be provided as image frames annotated by clinical experts as being optimal for metrics measurements.

Providing the selection by artificial intelligence makes it possible to perform that selection locally and without explicit formulation of metrics of quality.

Alternatively, for training, the experts can manually measure the stenosis (ratio of smallest vessel diameter to reference size). The algorithm then processes each image of the sequence, and the preferred image will be the one for which the metrics are closest to the measures determined by the expert, thus building a database.

Finally, once this reference database has been built (sequences and ROI as inputs, preferred frame or reference measure as targeted output), a learning algorithm is trained to automatically perform the selection.

The quantitative analysis of the stenoses as visible in the angiograms yields a series of metrics that allow the clinician to analyze, discuss, and document an examination.

Thus, the practical evaluation of the QCA is accelerated. Upon selecting the frame, an extraction of the metrics can be provided.

In an example, the selected image frame is provided to the user.

The selected frame may be provided for documentation and reporting purposes. In an option, the frame is stored together with a computed metrics measurement characterizing the stenosis.

In an example, the selected image frame is displayed.

As an option, indicated in Fig. 4 with hashed lines, it is provided a step 216 of determining region of interest comprising the stenosis, before computing the predetermined stenosis-related measurement.

In an example of the method, it is provided the step of segmenting at least the part of the vascular structure of the selected image frame which part comprises the stenosis.

In an example of the method, predetermined metrics measurements relating to the stenosis are performed for the selected image frame. Further, the metrics measurements are provided.

In another option, the choice of the sequence of interest is provided and then the frame of interest is selected in the selected sequence. For further steps, the clinician has to click on the stenosis.

As an option, the selection is applied to other algorithms that would be applied on an image, i.e. where a frame is previously selected from a sequence in order to obtain an optimal local visibility. In a first option, the selection is provided in view of optimal calcium detection instead of the stenosis measurements. In a second option, the selection is provided in view of optimal bifurcation analysis instead of the stenosis measurements.

In an example, the selection of a best frame inside an already selected sequence is provided. In another example, the selection of a sequence is provided and a best frame inside the selected sequence is provided.

In an example, a framework selects from an angiogram the frame that is optimal for stenosis analysis, locally and without explicit formulation of metrics of quality. The algorithm takes as input one sequence and a region of interest that can be large, but separates the stenosis of interest from the others. It outputs the image of the sequence that will guarantee the stenosis quantification closest to the truth.

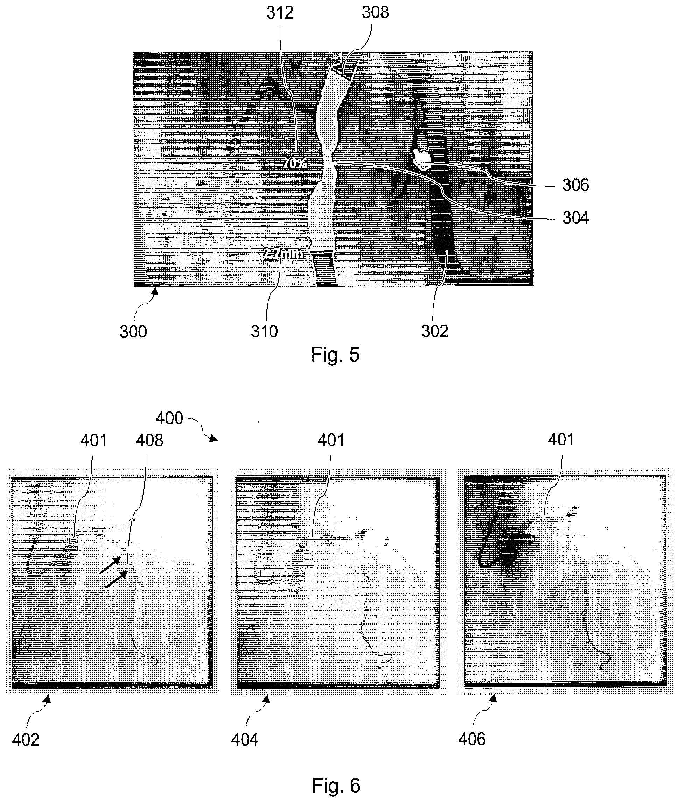

Fig. 5 shows an example of an angiogram 300 for quantitative coronary analysis. A part of a vascular structure 302 is visible in the angiogram 300. Fig. 5 shows an angiography image in which the vessels are at least partly filled with contrast agent to make the vascular structure 302 visible in the X-ray image. Upon identifying a stenosis 304, e.g. by manual interaction with a pointer 306, a ratio of a normal width part 308 and the smallest diameter of the stenosis con be determined. A value 310 of the normal width portion is indicated as well as a percentage 312 for the width relation.

The present invention targets on providing such suitable image, i.e. such frame that can be used for stenosis analysis.

Fig. 6 shows an example of three different images of an angiographic sequence 400 showing a vascular structure 401. A first image 402, or frame, is shown in the left part, a second image 404, or frame, is shown in the middle part and a third image 406, or frame, is shown in the right part. The third image 406 is provided with cluttering and faint contrast. The second image 404 is provided with cluttering. The first image 402 is best suited for stenosis analysis. Two arrows 408 indicate stenoses. The present invention provides selecting such best suitable image.

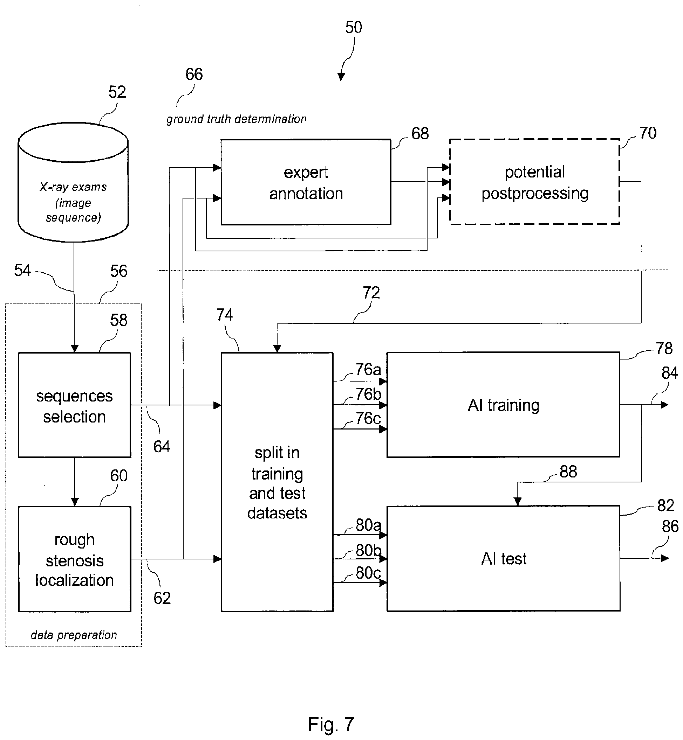

Fig. 7 shows a further example of a framework 50 for selecting an angiogram for stenosis analysis. X-ray exams, i.e. image sequences 52 are provided as data supply 54 to a first module 56 that provides data preparation thereof. The data that will serve as input data to the frame selection algorithm. In an option, from a collection of recorded X-ray exams (angiographic and fluoroscopic image sequences, potentially with some metadata), sequences suitable for stenosis analysis are selected in a selection step 58.

In an example, balloon inflations are detected that were visible on some fluoroscopies, e.g. by image processing. All the angiograms that were acquired from the same C-arm angulation before the first ballooning are interesting candidates: They show the stenosis before treatment from an angulation well suited for visualizing region of interest.

One can then select the first one, e.g. which was acquired for diagnosis purposes, the last one, e.g. which shows the landscape immediately before ballooning, or the most injected one.

An alternative is to learn the sequence selection process by artificial intelligence.

Within the data preparation 56, for each selected angiogram, an approximate position of the stenosis is determined, i.e. a rough stenosis localization 60 is provided. This allows then to focus on the lesion of interest, and to ignore other potential stenoses. This region of interest is expected to comprise the stenosis of interest, and excludes the other ones. Since in practice the stenosis are far from each other when there are many (otherwise they are considered one unique lesion), the ROI does not have to be accurate.

As an example, an automatic approximate localization uses the mask of the dilated balloon from the ballooning images as an indication of the position (and orientation) of the stenosis of interest in the angiographic image. The ballooning images may be (low dose) fluoroscopy images or other (normal or higher dose) X-ray images. The balloon position is not exactly the same than the stenosis position, since the cardiac and breathing status are probably different, and the table may have moved. Still, this information is most of the time sufficient to reject the other stenosis candidates.

As an output, localization 62 is provided. The option mentioned above provides as output an angio sequence 64.

Now, in the training phase, this data is associated with selected frame indices that will serve as ground truth in a ground truth association module 66. This step requires some annotations 68 carried out by experts.

In a first option, the experts directly determine which frame they prefer. More specifically, they are provided with a tool that displays the selected sequence, and ways to browse it (previous/next frame). They are simultaneously shown the region of interest, e.g. by plotting a closed curve over the sequence or by showing a binary mask in parallel to the sequence to annotate. The observer can thus retrieve where the region of interest is. The experts select the image that they judge preferable to perform the stenosis analysis. This qualitative formulation is clear for experts, while being close to impossible to formalize quantitatively.

In an alternative option, the experts can semi-automatically measure the stenosis severity, e.g. performing QCA. In that case, as an option, in a post-processing step 70, the stenosis analysis is run on each frame, and the one closest to the expert measure is selected. This second option also allows to associate each frame with a score (depending on how close the automatic estimation performed on that frame is close to the expert measure). Different artificial intelligence algorithms are then developed based on this different kind of ground truth, which attempts to estimate a frame quality score - to select the frame with the highest estimated score.

Finally, this set of data (angiographic sequences 64 with ROI 62) and ground truth (selected frames with selected frame index, or frame quality score - indicated with second arrow 72) is provided to a further split module 74 and is split into different sets. Typically a training set and a test set, e.g. with possibly an additional validation set, are provided.

The split module 74 thus provides a training angio sequence 76a, a training localization 76b and training selected frames 76c to an AI (artificial intelligence) training module 78. As an option, machine learning is provided in the algorithm training module 78.

The split module 74 thus further provides a test angio sequence 80a, a test localization 80b and test selected frames 80c to an AI (artificial intelligence) test module 82. As an option, machine learning is provided in the AI test module 82.

The output from the AI training module 78 is provided to a frame selection algorithm 84. The output from the AI test module 82 is provided to a frame selection algorithm evaluation 86. A third arrow 88 indicates that the result from the AI training module 78 can also be provided to the AI test module 82.

Artificial intelligence algorithms are trained on the training set, in order to learn ways to infer the ground truth from the input data, e.g. applying classification methods, like VGG-net, to associate each frame with a class, such as selected/rejected. In an example, feature vectors are extracted from each frame using identical convolution networks. The feature vectors are used to perform a classification (via recurrent networks for instance). If the ground truth is made of scores instead of indices, the classification schemes can be substituted with regression schemes. The trained network is applied to the test data to measure the quality of the selection algorithm.

In an option, recurrent networks to allow for sequences of flexible size. This is useful, for example, for applying AI at the level of the sequence, the size of which varies temporally.

Finally, once trained, the network will be applied to images for which we have no ground truth to perform inference - and determine which frame should be preferred to then get the most accurate stenosis measure.

The term "subject" may also be referred to as individual. The "subject" may further also be referred to as patient, although it is noted that this term does not indicate whether any illness or disease is actually present with the subject.

In an example, a computer program enabling a processor to carry out the method of one of the examples above is provided. In a further example, a computer program or program element for controlling an apparatus according to one of the examples above is provided, which program or program element, when being executed by a processing unit, is adapted to perform the method steps of one of the method examples above. In another example, a computer readable medium is provided having stored the program element of the example above.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for selecting an angiogram for stenosis analysis, comprising:
- an image sequence supply (12);
- a data processor (14); and
- an output (16);
wherein the image sequence supply is configured to provide a sequence of image frames representative of a region of interest of a vascular structure comprising one stenosis;
wherein the data processor is configured to select an image frame of the sequence of image frames that leads to best matching stenosis related measurements; wherein the selecting is based at least indirectly on at least one reference; and wherein the best matching relates at least indirectly to a maximized accuracy of the measurements; and
wherein the output is configured to provide the selected image frame.

2. Device according to claim 1, wherein, for selecting the image frame, the data processor comprises a machine learning algorithm that is configured to be trained with training datasets comprising pre-selected exemplary image frames comprising a stenosis along with some ground truth comprising at least one of the group of: preferred frame number as selected by an expert or reference measure.

3. Device according to claim 2, wherein, for selecting the image frame, the data processor with a trained machine learning algorithm is configured to provide the selection without further ground truth and/or further comparison with a predetermined reference.

4. Device according to claim 2 or 3, wherein, for training of the selecting the image frame, the data processor is configured to compute a predetermined stenosis-related measurement for at least two of the image frames of the sequence of images; and to compare the computed measurements with a reference measurement and to determine one of the computed measurements that is best matching with the reference measurement.

5. Device according to one of the preceding claims, wherein the data processor is configured to determine a region of interest comprising the stenosis before the predetermined stenosis-related measurement is computed.

6. Device according to one of the preceding claims, wherein the data processor is configured to perform, for the selected image frame, predetermined metrics measurements relating to the stenosis; and
wherein the output is configured to provide the measurements.

7. Device according to one of the preceding claims, wherein the data processor is configured to segment at least the part of the vascular structure of the selected image frame which part comprises the stenosis.

8. A system (100) for stenosis analysis, comprising:
- a device (10) for selecting an angiogram for stenosis analysis according to one of the preceding claims; and
- an X-ray imaging arrangement (102);
wherein the X-ray imaging arrangement is configured to provide the sequence of image frames.

9. A method (200) for selecting an angiogram for stenosis analysis, comprising the following steps:
- providing (202) a sequence of image frames representative of a region of interest of a vascular structure comprising one stenosis;
- selecting (204) an image frame of the sequence of image frames that leads to best matching stenosis related measurements; wherein the selecting is based at least indirectly on at least one reference; and wherein the best matching relates at least indirectly to a maximized accuracy of the measurements; and
- providing (206) the selected image frame.

10. Method according to claim 9, wherein for selecting the image frame, a machine learning algorithm is trained with training datasets comprising pre-selected exemplary image frames comprising a stenosis along with some ground truth comprising at least one of the group of: preferred frame number as selected by an expert or reference measure.

11. Method according to claim 10, wherein for selecting the image frame, the trained machine learning algorithm is providing the selection without further ground truth and/or further comparison with a predetermined reference.

12. Method according to claim 9, 10 or 11, wherein for training of the selecting the image frame it is provided the steps of:
- computing (208) a predetermined stenosis-related measurement for at least two of the image frames of the sequence of images; and
- comparing (210) the computed measurements with a reference measurement and determining (212) one of the computed measurements that is best matching with the reference measurement.

13. A computer program enabling a processor to carry out the method of one of the claims 9 to 12.

14. A computer readable medium having stored the program element of claim 13.
